# EUROPÄISCHE PATENTSCHRIFT

(11) **EP 2 135 586 B1**
(45) Veröffentlichungstag und Bekanntmachung des Hinweises auf die Patenterteilung: **31.08.2011**
(21) Anmeldenummer: 09007906.2
(22) Anmeldetag: 17.06.2009
(51) Int. Cl.: A61F 2/44

(54) **Implantat zum Einsetzen zwischen Wirbelkörper der Wirbelsäule**
Implant for inserting between vertebrae of the spine
Implant destiné à l'intégration entre des vertèbres de la colonne vertébrale

(30) Priorität: 18.06.2008 DE 102008028589
(43) Veröffentlichungstag der Anmeldung: 23.12.2009
(73) Patentinhaber: Rüger, Florian, 38723 Seesen (DE)
(72) Erfinder: Rüger, Florian, 38723 Seesen (DE)
(74) Vertreter: Callies, Rainer Michael

(56) Entgegenhaltungen:
- EP-A- 0 950 389
- FR-A- 2 734 148
- US-A1- 2005 015 152
- US-A1- 2005 251 260
- MANFRED MEISSNER: 1988, VEB VERLAG TECHNIK , BERLIN , XP002544977 * Seite 8 * * Seite 109 - Seite 114 *

## Beschreibung

Die Erfindung bezieht sich auf Implantate nach dem Oberbegriff des Anspruchs 1. Bei Frakturen von einem oder mehreren Wirbelkörpern im Bereich des Übergangs von der Brust- zur Lendenwirbelsäule (thorakolumbalen Übergangs) wird im Falle einer instabilen Fraktur eine dorsale Stabilisierung mittels eines Stangen-Schrauben-Systems (Fixateur Interne Systems) zum Verbinden von Wirbeln vorgenommen. Dabei werden Wirbelkörper, die dem frakturierten Wirbel anlegen, transpedikulär von dorsal durchschraubt und über Führungsstäbe miteinander verbunden, wodurch sich eine funktionelle Fusion des gesamten miteinander verschraubten Wirbelsäulensegments ergibt.

In einem zweiten operativen Eingriff kann, wenn ein Bandscheibenschaden vorliegt oder weiterhin eine Instabilität besteht, eine weitere Stabilisierung über ein oben genanntes Implantat, das nach einem Entfernen eines Teils der Wirbelsäule ventral in die Wirbelsäule eingesetzt wird, erreicht werden.

Auch wenn in der Regel nach einer gewissen Zeit das Stangen-Schrauben-System zur Entlastung des Patienten von Metall wieder entfernt wird, ist durch das Einsetzen eines herkömmlichen Implantats eine Versteifung des betroffenen Wirbelsäulenbereichs gegeben. Eine Versteifung eines Wirbelsäulenbereichs führt jedoch zu einer ungedämpften Kraftweiterleitung in die Wirbel und Bandscheiben, die caudal des versteiften Wirbelsäulenbereichs gelegen sind. In Folge der in dem versteiften Bereich nicht mehr vorhandenen Dämpfung entsteht eine Überbeanspruchung der caudal gelegenen Wirbel und Bandscheiben, indem Kräfte aus dem versteiften Bereich ungedämpft weitergeleitet werden. So hat sich postoperativ bei einer bedeutenden Anzahl von Patienten eine Überlastung des direkt an den operierten Bereich anschließenden Wirbelsäulensegments gezeigt, mit der Folge von Rückenbeschwerden und einer erhöhten Anzahl von Bandscheibenvorfällen. Kräfte, die auf ein Implantat wirken, sind *in vivo* gemessen worden (Rohlmann A. et al., "Loads on a Telemeterized Vertebral Body Replacement Measured in Two Patients", Spine 2008, Vol. 33, Nr. 11: 1170-9).

Es kann herkömmlich durchaus beabsichtigt sein, in dem geschädigten und durch ein Implantat teilweise ersetzten Wirbelsäulenbereich eine möglichst starke Versteifung zu erreichen, um eine stabile Fusion des Implantats mit den benachbarten Wirbelkörpern zu erreichen.

Ein Stangen-Schrauben-System ist aus der DE 103 27 358 A1 bekannt. Es weist eine gewisse Biegsamkeit auf, um Bewegungen einer gesunden Wirbelsäule teilweise nachzuahmen.

Ein herkömmliches Implantat ist beispielsweise aus der EP 1 872 748 A1 bekannt. Nachdem dieses Implantat in die Wirbelsäule eingesetzt und in seiner Höhe eingestellt und fixiert worden ist, ermöglicht es zwar aufgrund einer Lagerplatte mit einem gekrümmten Lagersitz den Erhalt von Beweglichkeit der betroffenen Wirbelsäulensegmente, jedoch keine Dämpfung von vertikal auf die Wirbelsäule wirkenden Kraftstößen.

Ein gattungsgemäßes Implantat ist aus der EP 0 950 389 A2 bekannt. Bei diesem handelt es sich jedoch um ein Fusionsimplantat. Fusionsimplantate fixieren die benachbarten Wirbelkörper so relativ zueinander, dass die beiden Wirbelkörper im Laufe der Zeit durch eine Brücke aus Knochensubstanz dauerhaft miteinander verbunden werden. Es ist somit eine Versteifung durch eine knöcherne Brücke zwischen den benachbarten Wirbelkörpern vorgesehen, weshalb auch hier eine Dämpfung von Kraftstößen nicht gegeben ist.

Der Erfindung liegt die Aufgabe zugrunde, ein gattungsgemäßes Implantat zur Verfügung zu stellen, mit dem eine Überbeanspruchung von caudal zu dem Implantat gelegenen Wirbeln und Bandscheiben verringert wird und bei dem ein maximaler Federweg gegeben ist.

Die Aufgabe wird durch die Merkmale des Anspruchs 1 bzw. des Anspruchs 3 gelöst. Das jeweilige Implantat dient zum Einsetzen zwischen Wirbelkörper der Wirbelsäule als Platzhalter für Wirbelkörper und Bandscheiben, die aus der Wirbelsäule entfernt worden sind, oder für Wirbelteile und Bandscheiben, die aus der Wirbelsäule entfernt worden sind. Das so eingesetzte Implantat ist also stets auch Platzhalter für mindestens eine entfernte Bandscheibe. Grund für die Entfernung der Wirbelkörper kann eine Wirbelkörperdestruktion durch eine Fraktur, eine Entzündung oder einen Tumor gewesen sein. Das Implantat weist ein erstes Implantatteil und ein zweites Implantatteil auf. Beide Implantatteile sind in Richtung ihrer koaxial zueinander angeordneten Längsachsen so gegeneinander verschiebbar, dass sich die Höhe oder anders ausgedrückt die Länge des Implantats verändert. Die beiden Implantatteile sind durch eine Feder miteinander verbunden, wobei auch ein Federsystem, das mehrere Federn aufweisen kann, möglich ist. Die Feder ist derartig ausgelegt, dass sie einem Verschieben der Implantatteile gegeneinander, bei dem eine Verringerung der Implantathöhe stattfindet, eine Federkraft entgegensetzt. Bei einem genannten Wirbelteil, für welches das Implantat als Platzhalter eingesetzt werden kann, kann es sich insbesondere um einen sich über die gesamte Höhe eines Wirbelkörpers erstreckenden Teil des Wirbelkörpers handeln.

Die Feder bzw. das Federsystem ist zwischen einer nach innen gerichteten Bodenfläche des ersten Implantatteils und einem unteren Ende des zweiten Implantatteils angeordnet, wobei das zweite in das erste Implantatteil hineinragt und innerhalb von diesem verschiebbar ist. Auf diese Weise kann der Vorteil einer guten Stabilität in Längsrichtung und einer gleichzeitigen Dämpfungswirkung in dieser Richtung kombiniert werden.

Die Feder ist eine Ringfeder. Diese Ringfeder weist bei dem Implantat gemäß Anspruch 1 zwei Innenringe und einen zwischen - vorzugsweise mittig zu - den Innenringen angeordneten Außenring auf, wobei die beiden Innenringe jeweils mit dem Außenring über Berührungsflächen, die vorzugsweise konisch sind, in Kontakt sind und ineinandergreifen. Dabei wird das Material der Innenringe auf Druck und das Material des Außenrings auf Zug beansprucht, so dass auf kleinstem Raum Kräfte aufgenommen werden können.

Vorzugsweise sind sowohl die beiden Innenringe als auch der Außenring nicht geschlossen, sondern durch einen Schlitz unterbrochen. Dies hat den Vorteil, dass sich die Durchmesser der Ringe leichter verändern als im Falle geschlossener Ringe. Da die Kräfte, die in dem entsprechenden Wirbelsäulenbereich wirken, absolut betrachtet eher gering sind, sind solche nicht geschlossenen Innen- bzw. Außenringe vorteilhaft. Möglich ist aber auch, alle oder einen Teil der verwendeten Ringe geschlossen vorzusehen.

Die Ringfeder liegt mit dem oberen und dem unteren Innenring lediglich gegen die beiden Implantatteile an, also gegen eine innere Bodenfläche des ersten Implantatteils und gegen ein unteres Ende des zweiten Implantatteils. Vorzugsweise liegt die Ringfeder gegen eine äußere Bodenfläche des zweiten Implantatteils an. Dadurch, dass die Ringfeder nach dem Einsetzen des Implantats - im Zustand einer Überstreckung des Wirbelsäulenbereichs - unter Vorspannung ist, unterliegt die Ringfeder keinem Spiel. Andererseits ist aber gewährleistet, dass sich die beiden Innenringe in ihrem Durchmesser verändern können.

Die Ringfeder ist bei dem Implantat gemäß Anspruch 1 so ausgelegt, dass ein maximaler Federweg dadurch gegeben ist, dass der Außenring mit einer Außenfläche gegen eine Wandungsinnenfläche des ersten Implantatteils anschlägt.

Die Ringfeder weist bei dem Implantat gemäß Anspruch 3 zwei Außenringe und einen - vorzugsweise mittig - zwischen den beiden Außenringen angeordneten Innenring auf. Die Funktionsweise einer solchen Ringfeder entspricht der Funktionsweise der vorstehend beschriebenen Ringfeder. Unterschiede bestehen darin, dass ein maximaler Federweg durch einen Anschlag einer jeweiligen Außenfläche der Außenringe gegen eine Wandungsinnenfläche des ersten Implantatteils gegeben ist.

Bei den erfindungsgemäßen Implantaten gemäß Anspruch 1 bzw. Anspruch 3 ist, wenn sie in die Wirbelsäule eingebracht sind, aufgrund der Feder eine Federung im frakturierten Wirbelsäulenbereich gegeben. Dabei ahmt die Federwirkung die physiologische Federung einer Bandscheibe nach. Auf diese Weise ist eine im Vergleich zu herkömmlichen Implantaten geringere Beanspruchung des caudal an das Implantat anliegenden Wirbelkörpers bzw. der sich caudal anschließenden Wirbelkörper und Bandscheiben bewirkt. Die positive Folge ist, dass bei entsprechend operierten Patienten weniger häufig Rückenbeschwerden und Bandscheibenvorfälle, die durch das eingesetzte Implantat bedingt sind, auftreten. Ein wesentlicher Vorteil der Verwendung der erfindungsgemäßen Implantate kann auch bereits in dem Umstand bestehen, dass Rückenbeschwerden oder Bandscheibenvorfälle erst nach einem im Vergleich zu herkömmlichen Implantaten längeren Zeitraum auftreten.

Wenn Patienten vor dem Einsetzen des Implantats ein Stangen-Schrauben-System erhalten haben, was möglicherweise auch in einem gewissen geringen Maße federbar ist, kommt ihnen die Federwirkung des Implantats in vollem Umfang dann zugute, wenn das Stangen-Schrauben-System entfernt wird.

Es kann erfingdungsgemäß ferner vorgesehen sein, dass die Implantate in ihrer Höhe einstellbar sind. Dazu kann eines der beiden Implantatteile, vorzugsweise das zweite Implantatteil, welches in das erste hineinragt, zwei Bestandteile aufweisen, die über ein Innengewinde und ein Außengewinde miteinander in Eingriff sind, wobei eine Arretierung möglich ist. Die Arretierung kann beispielsweise durch einen Stift erfolgen, der in einen Längsschlitz des gedrehten Bestandteils eingreift, oder durch eine Sicherungsschraube.

Eine Höhenverstellbarkeit bzw. eine Einstellbarkeit der gewünschten Distraktion ist vorteilhaft beim Einsetzen des Implantats, da dann eine Überstreckung des Wirbelsäulenbereichs nicht erforderlich ist. Ferner kann durch die nach dem Einsetzen des Implantats erfolgende Justierung der Implantathöhe die Vorspannung der Feder bzw. des Federsystems eingestellt werden.

Die Implantate sind vorzugsweise vollständig aus Titan hergestellt. Dieses Material ist vorteilhaft bei möglicherweise stattfindenden kernspintomographischen Untersuchungen.

Im Folgenden wird die Erfindung anhand eines Ausführungsbeispiels näher erläutert, wobei auf die Figuren Bezug genommen wird. Es zeigt:

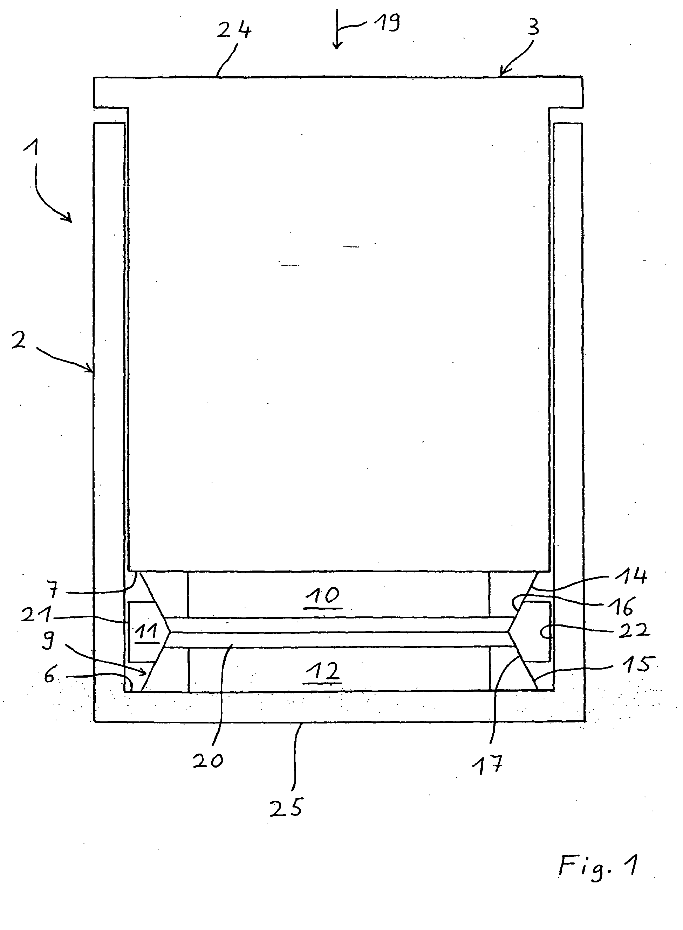

Fig. 1 eine schematische Darstellung eines Implantats mit einer Ringfeder im Längsschnitt.

Das gezeigte Implantat ist mit dem Bezugszeichen 1 bezeichnet. Es weist ein erstes bzw. - beim Einsetzen in caudaler Richtung gesehen - unteres Implantatteil 2 auf, das im Wesentlichen zylinderförmig ist. In das untere Implantatteil ragt ein zweites bzw. oberes Implantatteil 3 hinein.

Das obere Implantatteil 3 ist stempelartig und verschiebbar innerhalb des unteren Implantatteils 2 angeordnet.

Zwischen eine innere Bodenfläche 6 des unteren Implantatteils 2 und eine äußere Bodenfläche 7 des oberen Implantatteils 3 ist eine Ringfeder 9 gelegt. Die Ringfeder 9 weist einen oberen Innenring 10, einen mittleren Außenring 11 und einen unteren Innenring 12 auf.

Die Innenringe 10, 12 sind nicht an dem Implantatteil 2, 3 befestigt, sondern liegen an sich lose gegen die Bodenflächen 6, 7 an. Wenn die Ringfeder 9 vorgespannt ist, wie dies bei dem gezeigten Zustand der Ringfeder 9 der Fall ist, kann sich die Ringfeder 9 jedoch nicht seitlich verschieben.

Sowohl die Innenringe 10, 12 als auch der Außenring 11 sind nicht geschlossen, sondern weisen einen (nicht gezeigten) Schlitz auf. Die Innenringe 10, 12 weisen jeweils eine konische Berührungsfläche 14 bzw. 15 auf. Der Außenring 11 weist ebenfalls konisch geformte Berührungsflächen 16 und 17 auf, die in Kontakt mit den Berührungsflächen 14, 15 der Innenringe 10, 12 sind.

Die Federkraft der Ringfeder 9 ergibt sich aus der Verformbarkeit der Innenringe 10, 12 und des Außenrings 11 hinsichtlich einer Veränderung ihres Durchmessers sowie aus der Reibung zwischen den Berührungsflächen 14 bis 17.

Wenn von oben eine Kraft auf das obere Implantatteil 3 wirkt, wie dies durch Pfeil 19 angedeutet ist, gleiten die Berührungsflächen 14, 15 und 16, 17 relativ zueinander, wobei sich der Durchmesser der Innenringe 10, 12 verkleinert, während sich der Durchmesser des Außenrings 11 vergrößert. Dabei verkleinert sich ein Zwischenraum 20 zwischen den beiden Innenringen 10, 12. Eine Bewegung des oberen Implantatteils 3 gemäß Pfeil 19 findet dadurch eine Grenze, dass eine Außenfläche 21 des Außenrings 11 gegen eine Wandungsinnenfläche 22 des unteren Implantatteils 2 stößt.

Eine obere freie Auflagefläche 24 des oberen Implantatteils 3 und eine untere freie Auflagefläche 25 des unteren Implantatteils 2 weisen vorzugsweise jeweils eine Reihe von Rastzähnen (nicht dargestellt) auf, die mit einer Schneide versehen sein können. Diese Rastzähne dienen dazu, das Implantat 1 in den angrenzenden Wirbelkörpern zu verankern, so dass ein seitliches Verschieben des Implantats 1 gegenüber den Wirbelkörpern verhindert ist.

Alternativ könnte die Ringfeder 9 auch zwei Außenringe und einen mittig zwischen den beiden Außenringen angeordneten Innenring aufweisen. Dann wäre ein maximaler Federweg durch einen Anschlag einer jeweiligen Außenfläche der Außenringe gegen die Wandungsinnenfläche 22 des unteren Implantatteils 2 gegeben.

Es könnte ferner vorgesehen sein, dass das Implantat 1 in seiner Höhe einstellbar ist. Dazu kann eines der beiden Implantatteile 2 und 3, vorzugsweise das obere Implantatteil 3, zwei Bestandteile aufweisen, die über ein Innengewinde und ein Außengewinde miteinander in Eingriff sind, wobei eine Arretierung möglich ist. Die Arretierung kann beispielsweise durch einen Stift erfolgen, der in einen Längsschlitz des gedrehten Bestandteils eingreift, oder durch eine Sicherungsschraube.

## Patentansprüche

1. Implantat (1) zum Einsetzen zwischen Wirbelkörper der Wirbelsäule als Platzhalter für aus der Wirbelsäule entfernte Wirbelkörper und Bandscheiben oder für aus der Wirbelsäule entfernte Wirbelteile und Bandscheiben, wobei das Implantat (1) ein erstes Implantatteil (2) und ein zweites Implantatteil (3) aufweist, welche in Richtung ihrer koaxialen Längsachsen unter Höhenänderung des Implantats (1) gegeneinander verschiebbar sind,
wobei die beiden Implantatteile (2,3) durch mindestens eine Feder (9) miteinander verbunden sind, welche einem mit einer Verringerung der Implantathöhe einhergehenden Verschieben der Implantatteile (2,3) gegeneinander eine Federkraft entgegensetzt,
**dadurch gekennzeichnet, dass** die Feder eine Ringfeder (9) ist und zwischen einer inneren Bodenfläche (6) des ersten Implantatteils (2) und einem unteren Ende (7) des zweiten Implantatteils (3), das in das erste Implantatteil (2) hineinragt, angeordnet ist,
dass die Ringfeder (9) zwei Innenringe (10,12), die jeweils an der inneren Bodenfläche (6) des ersten Implantatteils (2) bzw. an dem unteren Ende (7) des zweiten Implantatteils (3) anliegen, und einen Außenring (11), der zwischen den Innenringen (10,12) angeordnet ist, aufweist
und dass das Implantat (1) derartig ausgelegt ist, dass der Außenring (11) eine Begrenzung seiner möglichen Durchmesserausdehnung durch einen Anschlag einer Außenring-Außenfläche (21) gegen eine Wandungsinnenfläche (22) des ersten Implantatteils (2) findet.

2. Implantat (1) nach Anspruch 1,
**dadurch gekennzeichnet, dass** einer der Innenringe (10) an einer äußeren Bodenfläche (7) des zweiten Implantatteils (3) anliegt.

3. Implantat zum Einsetzen zwischen Wirbelkörper der Wirbelsäule als Platzhalter für aus der Wirbelsäule entfernte Wirbelkörper und Bandscheiben oder für aus der Wirbelsäule entfernte Wirbelteile und Bandscheiben, wobei das Implantat ein erstes Implantatteil (2) und ein zweites Implantatteil (3) aufweist, welche in Richtung ihrer koaxialen Längsachsen unter Höhenänderung des Implantats gegeneinander verschiebbar sind,
wobei die beiden Implantatteile (2,3) durch mindestens eine Feder miteinander verbunden sind, welche einem mit einer Verringerung der Implantathöhe einhergehenden Verschieben der Implantatteile (2,3) gegeneinander eine Federkraft entgegensetzt,
**dadurch gekennzeichnet, dass** die Feder eine Ringfeder ist und zwischen einer inneren Bodenfläche (6) des ersten Implantatteils (2) und einem unteren Ende (7) des zweiten Implantatteils (3), das in das erste Implantatteil (2) hineinragt, angeordnet ist,
dass die Ringfeder zwei Außenringe, die jeweils an der inneren Bodenfläche (6) des ersten Implantatteils (2) bzw. an dem unteren Ende (7) des zweiten Implantatteils (3) anliegen, und einen Innenring, der zwischen den Außenringen angeordnet ist, aufweist
und dass das Implantat derartig ausgelegt ist, dass die Außenringe eine Begrenzung ihrer Durchmesserausdehnung durch einen Anschlag einer Außenring-Außenfläche gegen eine Wandungsinnenfläche (22) des ersten Implantatteils (2) finden.

4. Implantat nach Anspruch 3,
**dadurch gekennzeichnet, dass** einer der Außenringe (10) an einer äußeren Bodenfläche (7) des zweiten Implantatteils (3) anliegt.

5. Implantat (1) nach einem der vorhergehenden Ansprüche,
**dadurch gekennzeichnet, dass** es in seiner Höhe **dadurch** einstellbar ist, dass eines der beiden Implantatteile (2,3) zwei Bestandteile aufweist, die über ein Innengewinde und ein Außengewinde miteinander in Eingriff sind und mittels eines Arretiermittels relativ zueinander fixiert werden können.

## Claims

1. Implant (1) for insertion between vertebral bodies of the spine as a placeholder for vertebral bodies and intervertebral disks which have been removed from the spine, or for spinal parts and intervertebral disks which have been removed from the spine, wherein the implant (1) comprises a first implant part (2) and a second implant part (3) which are displaceable with respect to each other in the direction of their coaxial longitudinal axes with the height of the implant (1) being changed,
wherein the two implant parts (2,3) are connected together by means of at least one spring (9) which opposes a displacement of the implant parts (2,3) with respect to each other, which is associated with a reduction in the implant height, by means of a spring force,
**characterised in that** the spring is an annular spring (9) and is disposed between an inner base surface (6) of the first implant part (2) and a lower end (7) of the second implant part (3) which protrudes into the first implant part (2),
the annular spring (9) comprises two inner rings (10, 12) which each lie against the inner base surface (6) of the first implant part (2) or against the lower end (7) of the second implant part (3), and comprises an outer ring (11) which is disposed between the inner rings (10, 12),
and the implant (1) is designed in such a manner that the outer ring (11) is limited in terms of its possible increase in diameter by virtue of the fact that an outer ring outer surface (21) abuts against a wall inner surface (22) of the first implant part (2).

2. Implant (1) as claimed in claim 1,
**characterised in that** one of the inner rings (10) lies against an outer base surface (7) of the second implant part (3).

3. Implant for insertion between vertebral bodies of the spine as a placeholder for vertebral bodies and intervertebral disks which have been removed from the spine, or
for spinal parts and intervertebral disks which have been removed from the spine, wherein the implant comprises a first implant part (2) and a second implant part (3) which are displaceable with respect to each other in the direction of their coaxial longitudinal axes with the height of the implant being changed,
wherein the two implant parts (2,3) are connected together by means of at least one spring which opposes a displacement of the implant parts (2,3) with respect to each other, which is associated with a reduction in the implant height, by means of a spring force,
**characterised in that** the spring is an annular spring and is disposed between an inner base surface (6) of the first implant part (2) and a lower end (7) of the second implant part (3) which protrudes into the first implant part (2),
the annular spring comprises two outer rings which each lie against the inner base surface (6) of the first implant part (2) or against the lower end (7) of the second implant part (3), and comprises an inner ring which is disposed between the outer rings,
and the implant is designed in such a manner that the outer rings are limited in terms of their increase in diameter by virtue of the fact that an outer ring outer surface abuts against a wall inner surface (22) of the first implant part (2).

4. Implant as claimed in claim 3,
**characterised in that** one of the outer rings (10) lies against an outer base surface (7) of the second implant part (3).

5. Implant (1) as claimed in any one of the preceding claims,
**characterised in that** it can be adjusted in terms of its height by virtue of the fact that one of the two implant parts (2,3) comprises two components which are in engagement with each other via an internal thread and an external thread and can be fixed relative to each other by means of a locking means.

## Revendications

1. Implant (1) destiné à être implanté entre les vertèbres de la colonne vertébrale sous forme de bouche-trou pour des vertèbres et des disques intervertébraux, ablatés de la colonne vertébrale ou pour des parties vertébrales et des disques intervertébraux, ablatés de la colonne vertébrale, ledit implant (1) comportant une première partie (2) et une deuxième partie (3), lesquelles peuvent être déplacées l'une vers l'autre dans la direction de leurs axes longitudinaux coaxiaux moyennant une variation de hauteur de l'implant (1),
les deux parties (2, 3) de l'implant étant reliées entre elles par au moins un ressort (9) qui oppose une force empêchant un déplacement des parties (2, 3) l'une vers l'autre, lequel va de pair avec une diminution de la hauteur de l'implant,
**caractérisé en ce que** le ressort est un ressort annulaire (9) et est agencé entre une face intérieure (6) du fond de la première partie (2) et une extrémité inférieure (7) de la deuxième partie (3), qui s'engage dans la première partie (2),
**en ce que** le ressort annulaire (9) comporte deux bagues intérieures (10, 12), qui sont en appui respectivement contre la face intérieure (6) du fond de la première partie (2) et contre l'extrémité inférieure (7) de la deuxième partie (3), et une bague extérieure (11) qui est agencée entre les bagues intérieures (10, 12),
et **en ce que** l'implant (1) est dimensionné de telle sorte que la bague extérieure (11) trouve une limite à la dilatation possible de son diamètre du fait qu'une face extérieure (21) de la bague extérieure vient buter contre une face intérieure (22) de la paroi de la première partie (2) de l'implant.

2. Implant (1) selon la revendication 1, **caractérisé en ce que** l'une des bagues intérieures (10) est en appui contre une face extérieure (7) du fond de la deuxième partie (3) de l'implant.

3. Implant destiné à être implanté entre les vertèbres de la colonne vertébrale sous forme de bouche-trou pour des vertèbres et des disques intervertébraux, ablatés de la colonne vertébrale ou pour des parties vertébrales et des disques intervertébraux, ablatés de la colonne vertébrale, ledit implant (1) comportant une première partie (2) et une deuxième partie (3), lesquelles peuvent être déplacées l'une vers l'autre dans la direction de leurs axes longitudinaux coaxiaux moyennant une variation de hauteur de l'implant,
les deux parties (2, 3) de l'implant étant reliées entre elles par au moins un ressort qui oppose une force empêchant un déplacement des parties (2, 3) l'une vers l'autre, lequel va de pair avec une diminution de la hauteur de l'implant,
**caractérisé en ce que** le ressort est un ressort annulaire et est agencé entre une face intérieure (6) du fond de la première partie (2) et une extrémité inférieure (7) de la deuxième partie (3), qui s'engage dans la première partie (2),
**en ce que** le ressort annulaire comporte deux bagues extérieures, qui sont en appui respectivement contre la face intérieure (6) du fond de la première partie (2) et contre l'extrémité inférieure (7) de la deuxième partie (3), et une bague intérieure qui est agencée entre les bagues extérieures,
et **en ce que** l'implant (1) est dimensionné de telle sorte que les bagues extérieures trouvent une limite à la dilatation possible de leur diamètre du fait qu'une face extérieure des bagues extérieures vient buter contre une face intérieure (22) de la paroi de la première partie (2) de l'implant.

4. Implant selon la revendication 3, **caractérisé en ce que** l'une des bagues extérieures (10) est en appui contre une face extérieure (7) du fond de la deuxième partie (3) de l'implant.

5. Implant (1) selon l'une quelconque des revendications précédentes, **caractérisé en ce qu'**il peut être réglé en hauteur par le fait que l'une des deux parties (2, 3) de l'implant comporte deux parties constituantes, qui sont en prise l'une avec l'autre par l'intermédiaire d'un filetage intérieur et d'un filetage extérieur et qui peuvent être immobilisées l'une par rapport à l'autre par un moyen d'arrêt.
